# EUROPEAN PATENT APPLICATION

(11) **EP 3 085 685 A1**
(43) Date of publication of application: **26.10.2016**
(21) Application number: 14875937.6
(22) Date of filing: 14.01.2014
(51) Int. Cl.: C07C 49/717, A61K 31/122, A23K 10/40

(54) **BETA-HINOKITOL DERIVATIVE AND USES THEREOF IN PREPARATION OF SPECIAL ANTIBACTERIAL AGENT FOR ANIMAL AND ANTIMICROBIAL GROWTH PROMOTER FOR FEED**

(30) Priority: 31.12.2013 CN 201310751901
(71) Applicant: Guangzhou Insighter Biotechnology Co., Ltd, Guangzhou, Guangdong 510663 (CN)
(72) Inventor: PENG, Xianfeng, Guangzhou Guangdong 510663 (CN); QIN, Zonghua, Guangzhou Guangdong 510663 (CN)
(74) Representative: Walsh, Marie Goretti
(86) International application number: PCT/CN2014/070603
(87) International publication number: WO 2015/100799

(57) **Abstract**

The present invention discloses β-hinokitiol derivatives and application thereof in preparation of animal antibacterial agents and antibacterial growth promoters used in feed. β-hinokitiol derivatives, having a structural formula as shown in formula (I): wherein, R is n-propyl, n-pentyl, benzyl, sec-butyl, ethyl, n-butyl, p-chlorobenzyl, p-fluorobenzyl, n-octyl, 1-fluoropropyl or n-tetradecyl. The compounds of the present invention as shown in formula I show low toxicity or non-toxicity to poultry, is safer and has stronger growth promotion properties in comparison with all patented compounds at present, and therefore is more suitable to be used as growth promoters in feed. The present invention has a very good application prospect in cultivation industry.

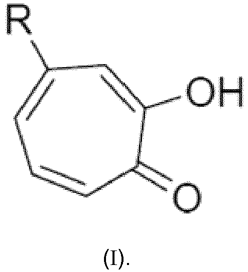

## Description

### Field of the Invention:

The present invention relates to the field of poultry cultivation, specifically to β-hinokitiol derivatives and application thereof in preparation of an animal antibacterial agent and an antibacterial growth promoter used in feed.

### Description of the prior art:

β-hinokitiol, chemical name: 2-hydroxy-4-isopropyl-1-cycloheptatrienone, is a natural edible essential oil ingredient, and has been used as a food additive for years in Japan.

US6387417B1 discloses activities of β-hinokitiol and complexes (2:1 or 3:1) inhibiting Enterococcus resistant to Vancomycin, and application thereof in treatment.

### Summary of the Invention:

The first object of the present invention is to provide β-hinokitiol derivatives which have broad-spectrum antibacterial activities and can be used as animal antibacterial agents and antibacterial growth promoters used in feed.

β-hinokitiol derivatives of the present invention have a structural formula as shown in formula (I).

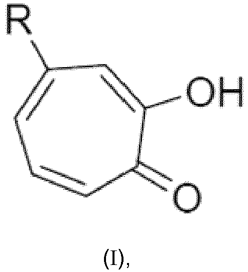

Wherein, R is n-propyl, n-pentyl, benzyl, sec-butyl, ethyl, n-butyl, p-chlorobenzyl, p-fluorobenzyl, n-octyl, 1-fluoropropyl or n-tetradecyl.

The specific structural formula is shown as below:

| | | |
|---|---|---|
| 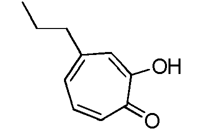 | 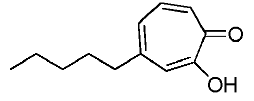 | 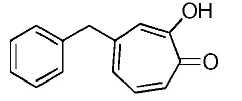 |
| Exact Mass: 164.08 IST_009_001 | Exact Mass: 192.12 IST_009_003 | Exact Mass: 212.08 IST_009_004 |
| | 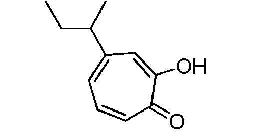 | 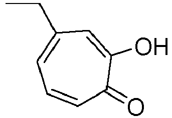 |
| | Exact Mass: 178.10 IST_009_008 | Exact Mass: 150.07 IST_009_018 |
| 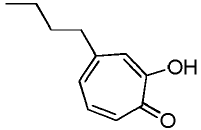 | 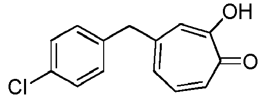 | 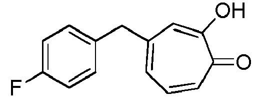 |
| Exact Mass: 178.10 IST_009_019 | Exact Mass: 246.04 IST_009_025 | Exact Mass: 230.07 IST_009_028 |
| 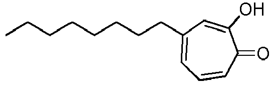 | 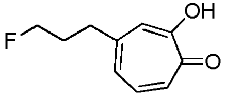 | 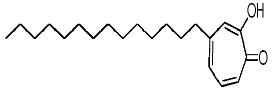 |
| Exact Mass: 234.16 IST_009_030 | Exact Mass: 182.07 IST_009_031 | Exact Mass: 318.26 IST_009_033 |

Through experiments, it has been found that the β-hinokitiol derivatives of the present invention have broad-spectrum antibacterial activities, show high inhibitory activities on major animal pathogenic bacteria (for example, clostridium perfringens, staphylococcus aureus, escherichia coli, salmonella, riemerella anatipestifer, haemophilus parasuis, vibrio and streptococcus), and in particular that, the antibacterial activities of additives such as IST_009_001 and IST_009_003 are better than those of the lead compound IST_001_002 (β-hinokitiol). Besides, through broiler feeding experiments, it has been found that β-hinokitiol derivatives such as IST_009_001 and IST_009_003 can enhance the weight gain and the feed conversion efficiency of the experimental broilers. Through weaned pig feeding experiments, it has been found that adding IST_009_001 in feeds can obviously reduce the diarrhea rate of the experimental pigs, and the effect of IST_009_001 is better than that of IST_001_002.

Therefore, the second object of the present invention is to provide applications of any one of the above-mentioned β-hinokitiol derivatives in preparation of animal antibacterial agents and antibacterial growth promoters used in feed.

The animals include pigs, chickens, ducks, geese, beef cattle, dairy cattle, sheep, fish, shrimp, foxes, martens or raccoon dogs in all growth stages.

Dosage of the β-hinokitiol derivative in animal feeds is 0.1 ∼200ppm.

When the animal antibacterial agents are used to treat diseases caused by infection with various animal sensitive bacteria, the dosage is 1-30mg/kg (body weight).

The compounds of the present invention as shown in formula I show low toxicity or non-toxicity to poultry, is safer and has stronger growth promotion properties in comparison with all patented compounds at present, and therefore is more suitable to be used as a growth promoters used in feed. The present invention has a very good application prospect in cultivation industry.

### Detailed Description of the Embodiments

The present invention is described in further detail with reference to embodiments which shall not be regarded as limits to the present invention.

11 β-hinokitiol derivatives of the present invention have structural formulas as shown below:

| | | |
|---|---|---|
| 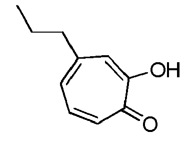 | 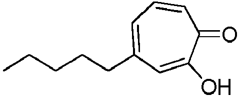 | 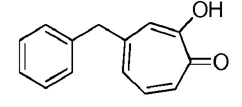 |
| Exact Mass: 164.08 IST_009_001 | Exact Mass: 192.12 IST_009_003 | Exact Mass: 212.08 IST_009_004 |
| | 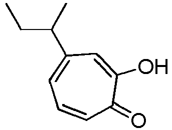 | 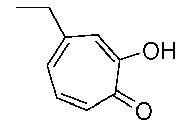 |
| | Exact Mass: 178.10 IST_009_008 | Exact Mass: 150.07 IST_009_018 |
| 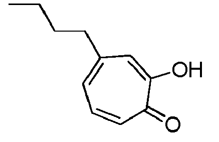 | 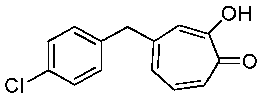 | 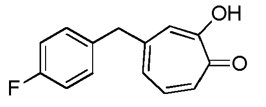 |
| Exact Mass: 178.10 IST_009_019 | Exact Mass: 246.04 IST_009_025 | Exact Mass: 230.07 IST_009_028 |
| 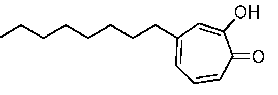 | 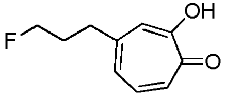 | 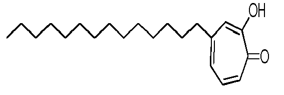 |
| Exact Mass: 234.16 IST_009_030 | Exact Mass: 182.07 IST_009_031 | Exact Mass: 318.26 IST_009_033 |

### Embodiment 1: Synthesis of IST_009_001 (2-hydroxy-4-propylcyclohepta-2,4,6-trienone)

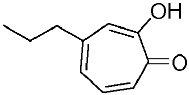

### Preparation of 2-hydroxy-4-propylcyclohepta-2,4,6-trienone

### Step 1:

At room temperature, to a 500mL three-necked flask was added 150ml of DMSO, stirred vigorously and purged with N₂ for 30 min. Potassium hydroxide (0.3mol, 1eq) was added and the mixture was continuously stirred for 0.5h. Then cyclopentadiene monomers (0.8∼1.2eq) was added dropwise within 30min and the resulting mixture was continuously stirred for 1h under N₂. Then, the mixture is cooled to about-10°C; the mixture of n-hexane (150ml) and bromopropane (0.8∼1.2eq) was added dropwise into the three-necked flask for about 1 h, with the temperature controlled to be not higher than 20°C. And then the reaction continued for 1h at room temperature; then, diluted hydrochloric acid was added dropwise to adjust the pH to be acidic; after the adjustment was completed, the mixture was stirred for 5-10min and then separated; the water layer was extracted one more time with 150ml of n-hexane; the combined organic layers was washed with 250ml of water twice until the water phase becomes neutral, dried over anhydrous sodium sulfate 40g, and filtrated, and then the filtrate (about 400ml) was added into a 1,000ml three-necked flask and let standing for 7-20h at a temperature of 20±2°C to achieve isomerization wherein gas chromatography (GC) was performed to determine the degree of isomerization, and n-propyl cyclopentadiene was obtained when the result is good.

### Step 2:

Under stirring, dichloroacetyl chloride (0.7∼1.5eq) was added into the n-hexane solution of the n-propyl cyclopentadiene; after the mixture was cooled to about -15∼10°C, triethylamine (0.8∼1.5eq) was added dropwise within 1h at the temperature controlled to be not higher than 15°C, and then the reaction proceeded for 1 h at a constant temperature; then, diluted hydrochloric acid was added dropwise to adjust the pH to be neutral or subacid; the solution was stirred for 5-10min and then separated, wherein concentration of the product in each of the resulting organic phase and the water phase was determined by thin layer chromatography (TLC, and with PE as a developing solvent). The organic phase was then subjected to rotary evaporation at 35°C to remove solvent, and the residual green liquid was dichlorobicycloketone.

### Step 3:

The dichlorobicycloketone prepared in the previous step, 125ml of tertiary butanol, 20ml of acetic acid and 25ml of water were added in sequence into a 1,000ml three-necked flask with a condenser pipe. The resulting reaction mixture was stirred at 75∼80°C; 85ml (98%, 0.675mol) triethylamine was added dropwise (for about 1 h), and then the mixture was kept in a constant temperature overnight. The reaction was monitored by TLC with petroleum ether (PE) as a developing solvent. At the completion of the reaction, the mixture was cooled to a temperature below 40°C, 300ml of dichloromethane was added into the mixture resulting in precipitation of a great amount of solids, and then diluted hydrochloric acid was added dropwise to adjust the pH to be acidic. The resulting mixture was stirred for 5-10min and then the organic phase was moved out. The precipitate in the flask was washed with 100ml of dichloromethane, and then the organic phase was moved out. Aqueous solution of potassium hydroxide (3M) was added to the combined organic phases, wherein the product is dissolved in the water phase, and a TLC test of the dichloromethane layer showed impurity spots but no product spot. Extraction and liquid separation were performed, the organic layer was removed, and the water phase was washed again with petroleum ether. The water phase was then added with petroleum ether, and then the mixed solution was added with aqueous solution of hydrochloric acid (3M) with stirring until the solution turns into acidic; the water phase was then removed, and the organic phase was washed with water three times. TLC of the organic phase (PE:EA=5:1) showed one spot. NMR result showed that the product is 2-hydroxy-4-propylcyclohepta-2,4,6-trienone, a structural formula of which is: 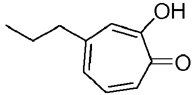 2-hydroxy-4-propylcyclohepta-2,4,6-trienone: δH (DMSO, 500 MHz) 7.271-7.313 (1 H,m), 7.144 (1H, s), 7.073-7.095 (1H, d, J=11), 6.912-6.932 (1H, d, J=10), 2.530-2.560(2H,m), 1.544-1.618 (2H,m), 0.867-0.896(3H,m).

### Embodiment 2: Synthesis of IST_009_003 (2-hydroxy-4-pentylcyclohepta-2,4,6-trienone)

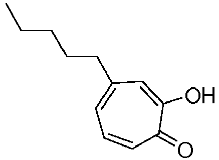

### Preparation of 2-hydroxy-4-pentylcyclohepta-2,4,6-trienone

### Step 1:

At room temperature, to a 500mL three-necked flask was added 150ml of DMSO, stirred vigorously and purged with N₂ for 30 min. Potassium hydroxide (0.3mol, 1eq) was added and the mixture was continuously stirred for 0.5h. Then cyclopentadiene monomers (0.8∼1.2eq) was added dropwise within 30min, and the mixture was continuously stirred for 1 h. Then, the mixture was cooled to about -10°C; a mixture of n-hexane (150ml) and 1-bromopentane (0.8∼1.2eq) was added dropwise into the three-necked flask within 1 h, at the temperature controlled to be not higher than 20°C. And then the reaction continued for 1h or more in a self-heating process; then, diluted hydrochloric acid was added dropwise to adjust the pH to be acidic; after the adjustment was completed, the mixture was stirred for 5-10min and then separated; the water layer was extracted one more time with 150ml of n-hexane; the combined organic layers was washed by 250ml of water twice until the water phase becomes neutral, dried over 40g of anhydrous sodium sulfate, and filtrated, and then the filtrate (about 400ml) was added into a 1,000ml three-necked flask and let standing for 7-20h at a temperature of 20±2°C to achieve isomerization wherein a GC was performed to determine the degree of isomerization, and n-pentyl cyclopentadiene was obtained.

### Step 2:

Under stirring, dichloroacetyl chloride (0.7∼1.5eq) was added into the n-hexane solution of the n-pentyl cyclopentadiene; after the mixture was cooled to about -15∼10°C, triethylamine (0.8∼1.5eq) was added dropwise for about 1 hr with the temperature controlled to be not greater than 15°C, and then the reaction proceeded for 1h at a constant temperature; then, diluted hydrochloric acid was added dropwise to adjust the pH to be neutral or subacid; the solution was stirred for 5-10min and then separated, wherein concentration of the product in each of the resulting organic phase and the water phase was determined by TLC (with PE as a developing solvent). The organic phase was then subjected to rotary evaporation at a temperature of 35°C to remove solvent, and the residual green liquid was dichlorobicycloketone.

### Step 3:

The dichlorobicycloketone prepared in the previous step, 125ml of tertiary butanol, 20ml of acetic acid and 25ml of water were added in sequence into a 1,000ml three-necked flask with a condenser pipe, stirred and heated to a temperature of 75∼80°C; 85ml (98%, 0.675mol) triethylamine was added dropwise (for about 1 h), and then the mixture was kept in a constant temperature overnight for reaction, and The reaction was monitored by TLC with petroleum ether (PE) as a developing solvent.. At the completion of the reaction, the mixture was cooled to a temperature below 40°C, 300ml of dichloromethane was added into the mixture resulting in precipitation of a great amount of solids, and then diluted hydrochloric acid was added dropwise to adjust the pH to be acidic. Then the solution was stirred for 5-10min and then separated, wherein TLC (developing solvent: EA:PA=1:5) result showed no presence of product in the resulting water layer. The organic phase (the lower layer) was dried over 40g of anhydrous sodium sulfate for 15min, filtrated and distilled at 35∼38°C to remove solvent. Residues were heated to 265°C and subjected to high vacuum distillation (with a rotary vane vacuum pump, above -0.098MPa); and fractions were collected at a temperature of 112∼116°C. At the completion of the distillation, nitrogen gas was introduced into the system. The fractions (about 8g) were added into 50ml petroleum ether under nitrogen protection wherein TLC (PE:EA=5:1) result shows two impurity spots above the production spot. The product was completely dissolved and the solution was placed in a refrigerator at a temperature of 4°C, wherein no solids separate out, and oil appears when it is cooled to -20°C.

The product was dissolved in petroleum ether, and then aqueous solution of potassium hydroxide (3M) was added into the mixture, wherein the product was dissolved in the water phase, and a TLC test of the dichloromethane layer showed impurity spots but no product spot. Extraction and liquid separation were performed, the organic layer was removed, and the water phase was washed again with petroleum ether. The water phase was then added with petroleum ether; and then the mixed solution was added with aqueous solution of hydrochloric acid (3M) with stirring until the solution turned into acidic; the water phase was then removed, and the organic phase was washed with water for three times. TLC of the organic phase (PE:EA=5:1) showed one spot. The organic phase was then subject to rotary evaporation to obtain a product. NMR result showed that the product was 2-hydroxy-4-pentylcyclohepta-2,4,6-trienone, a structural formula of which is: 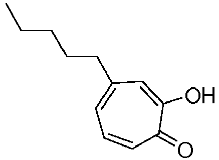 2-hydroxy-4-pentylcyclohepta-2,4,6-trienone: δH (DMSO, 500 MHz) 7.269-7.310 (1H, m), 7.142(1 H,s), 7.071-7.093 (1H, d, J=11), 6.912-6.932 (1H, d, J=10), 2.544-2.575(2H,m), 1.526-1.584(2H,m), 1.268-1.292(4H,m), 0.828-0.854(3H,m).

### Embodiment 3: Synthesis of IST_009_004

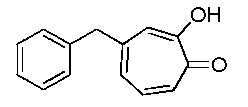

### Preparation of 4-benzyl-2-hydroxycyclohepta-2,4,6-trienone

### Step 1:

At room temperature, to a 500mL three-necked flask was added 150ml of DMSO, stirred and purged with N₂. Under the N₂ protection, potassium hydroxide (0.3mol, 1eq) was added and the mixture was continuously stirred for 0.5h. Then cyclopentadiene monomers (0.8∼1.2eq) was added dropwise within 30min, and the resulting mixture was continuously stirred for 1 h under the N₂ protection. Then, the mixture was cooled to about -10°C; a mixed solution of n-hexane (150ml) and benzyl bromide (0.8∼1.2eq) was added dropwise into the three-necked flask for about 1 h, with the temperature controlled to be not higher than 20°C. And then the reaction continued for 1h or more in a self-heating process; then, diluted hydrochloric acid was added dropwise to adjust the pH to be acidic; after the adjustment was completed, the mixture was fully stirred for 5-10min and then separated; the water layer was extracted one more time with 150ml of n-hexane; the combined organic layers were washed with 250ml of water twice until the water phase was neutral, dried over 40g of anhydrous sodium sulfate, and filtrated, and then the filtrate (about 400ml) is added into a 1,000ml three-necked flask and let standing for 7-20h at a temperature of 20±2°C to achieve isomerization wherein a GC was performed to determine the degree of isomerization, and benzyl cyclopentadiene was obtained.

### Step 2:

Under stirring, dichloroacetyl chloride (0.7∼1.5eq) was added into the n-hexane solution of the benzyl cyclopentadiene; the resulting mixture was cooled to about -15∼10°C, triethylamine (0.8∼1.5eq) was added dropwise for about 1 hr with the temperature controlled to be not greater than 15°C, and then the reaction proceeded for 1h at a constant temperature; then, diluted hydrochloric acid was added dropwise to adjust the pH to be neutral or subacid; the solution was stirred for 5-10min and then separated, wherein concentration of the product in each of the resulting organic phase and the water phase was determined by TLC (with PE as a developing solvent). The organic phase was then subjected to rotary evaporation at 35°C to remove solvent, and the residual green liquid was dichlorobicycloketone.

### Step 3:

The dichlorobicycloketone prepared in the previous step, 125ml of tertiary butanol, 20ml of acetic acid and 25ml of water were added in sequence into a 1,000ml three-necked flask with a condenser pipe and heated to a temperature of 75∼80°C with stirring; 85ml (98%, 0.675mol) triethylamine was added dropwise (for about 1 h), and then the resulting mixture was kept in a constant temperature overnight for reaction. The reaction was monitored by TLC with PE as a developing solvent. At the completion of the reaction, the mixture was cooled to a temperature below 40°C, 150ml of dichloromethane was added into the mixture, and then diluted hydrochloric acid (10ml of concentrated hydrochloric acid +200ml of water) was added dropwise to adjust the pH to be acidic. After the pH adjustment, the solution was stirred for 5-10min and then separated, wherein TLC (developing solvent: EA:PA=1:5) result showed no presence of product in the resulting water layer. To the organic phase (the lower layer) was added 200ml of water, followed by 150ml of aqueous solution of potassium hydroxide (3M) with stirring; and then was subject to extraction without separation. Haft of the upper layer was added into about 200ml of petroleum ether for extraction and separation. The water layer was washed for one time with petroleum ether. Then, the water layer was added with 200ml of petroleum ether, and diluted hydrochloric acid (3M) which was added into the mixture with stirring to adjust the pH to 1-2, for extraction and separation into a new water layer and a new organic layer, wherein solids separates out of the organic layer. The organic layer was added with a small amount of ethyl acetate to completely dissolve the solids, and then subject to extraction and separation into a new water layer and a new organic layer. The organic layer was subject to rotary evaporation until about 50ml thereof remains, placed in a refrigerator overnight to crystallize and filtrated to collect the crystal product. The crystals were washed with petroleum ether and thereafter 2.2g of product was obtained. NMR result showed that the product is 4-benzyl-2-hydroxycyclohepta-2,4,6-trienone, a structural formula of which is: 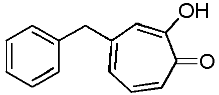 4-benzyl-2-hydroxycyclohepta-2,4,6-trienone: δH (DMSO, 500 MHz) 7.303-7.316 (3H, m), 7.269-7.283(2H, d,J=7), 7.211-7.239 (1H, m), 7.183 (1H, s), 7.066-7.088 (1H, d, J=11), 6.985-7.005 (1H,d,J=10), 3.952(2H,s).

### Embodiment 4: Synthesis of IST_009_008

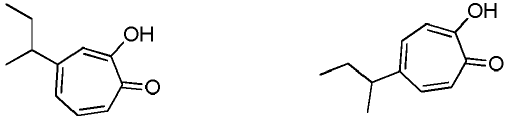

### Preparation of 4-(sec-butyl)-2-hydroxycyclohepta-2,4,6-trienone and 5-(sec-butyl)-2-hydroxycyclohepta-2,4,6-trienone

### Step 1:

At room temperature, to a 500mL three-necked flask was added 150ml DMSO, stirred and purged with N₂ for 30min to replace the air in the flask. Under the N₂ protection, potassium hydroxide (0.3mol, 1eq) was added and the mixture was continuously stirred for 0.5h. Then cyclopentadiene monomers (0.8∼ 1.2eq) was added dropwise within 30min, and the resulting mixture was continuously stirred for 1 h under the N₂ protection. Then, the mixture was cooled to about -10°C; a mixture of n-hexane (150ml) and 2-bromobutane (0.8∼1.2eq) was added dropwise into the three-necked flask for about 1h with the temperature controlled to be not higher than 20°C. And then the reaction continued for 1h or more in a self-heating process; then, diluted hydrochloric acid was added dropwise to adjust the pH to be acidic and the resulting mixture was stirred for 5-10min and then separated; the water layer was extracted one more time with 150ml of n-hexane; the combined organic layers was washed with 250ml of water twice until the water phase became neutral, dried over 40g of anhydrous sodium sulfate and filtrated. Then the resulting filtrate (about 400ml) was added into a 1,000ml three-necked flask and let standing for 7-20h at a temperature of 20±2°C to achieve isomerization wherein a GC was performed to determine the degree of isomerization, and sec-butyl cyclopentadiene was obtained.

### Step 2:

Under stirring, dichloroacetyl chloride (0.7∼1.5eq) was added into the n-hexane solution of the sec-butyl cyclopentadiene; after the mixture was cooled to about -15∼10°C, triethylamine (0.8∼1.5eq) was added dropwise for about 1hr with the temperature controlled to be not higher than 15°C, and then the reaction proceeded for 1h at a constant temperature; then, diluted hydrochloric acid was added dropwise to adjust the pH to be neutral or subacid; the solution was stirred for 5-10min and then separated, wherein concentration of the product in each of the resulting organic phase and the water phase was determined by TLC (with PE as a developing solvent). The organic phase was then subjected to rotary evaporation at a temperature of 35°C to remove solvent, and the residual green liquid was dichlorobicycloketone.

### Step 3:

The dichlorobicycloketone prepared in the previous step, 125ml of tertiary butanol, 20ml of acetic acid and 25ml of water were added in sequence into a 1,000ml three-necked flask with a condenser pipe, and the reaction mixture was heated to a temperature of 75∼80°C with stirring; 85ml (98%, 0.675mol) triethylamine was added dropwise (for about 1 h), and then the resulting mixture was kept in a constant temperature overnight, and the reaction was monitored by TLC with PE as a developing solvent. At the completion of the reaction, the mixture was cooled to a temperature below 40°C; 300ml of dichloromethane was added into the mixture resulting in precipitation of a great amount of solids. The solids were separated from the liquid, washed with petroleum ether, and then filtrated to obtain another organic phase. The combined organic phase was washed with 200mL of distilled water and subjected to rotary evaporation to remove the solvent. The residues were subject to decompression distillation and fractions of 100-111°C were collected with a vacuum degree of -0.099 and a bath temperature of 170-175°C.

The collected fractions were dissolved in 150ml of petroleum ether in a low-temperature reactor. The mixture was cooled from room temperature to -25°C so that white crystals was separated out, and then was subjected to filtration. Soon the solids melted, and the resulting oily substances were washed with petroleum ether and thereafter white solid appeared. NMR result showed that the white solid were 5-(sec-butyl)-2-hydroxycyclohepta-2,4,6-trienone. Then the filtrate was subjected to rotary evaporation to remove the solvent, and thereafter 4-(sec-butyl)-2-hydroxycyclohepta-2,4,6-trienone and 5-(sec-butyl)-2-hydroxycyclohepta-2,4,6-trienone were obtained.
4-(sec-butyl)-2-hydroxycyclohepta-2,4,6-trienone: δH (DMSO, 500 MHz) 7.302-7.344 (1 H, m), 7.127 (1H, s), 7.085-7.106 (1H, d, J=10.5), 6.097-6.927 (1H, d, J=10), 2.535-2.604 (1H, m), 1.503-1.561 (2H,m), 1.152-1.165(3H,d,J=6.5), 0.738-0.768(3H,m)
5-(sec-butyl)-2-hydroxycyclohepta-2,4,6-trienone: δH (DMSO, 500 MHz) 7.268-7.291 (2H,d,J=11.5), 7.167-7.191 (2H,d,J=12), 2.527-2.614 (1H, m), 1.477-1.584 (2H,m), 1.153-1.167(3H,d,J=7), 0.743-0.773(3H,m).

**Embodiment 5: Synthesis of IST_009_018** (4-ethyl-2-hydroxycyclohepta-2,4,6-trienone)

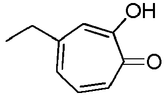

### Preparation of 4-ethyl-2-hydroxycyclohepta-2,4,6-trienone

### Step 1:

At room temperature, to a 500ml three-necked flask was added 150ml of DMSO, stirred and purged with N₂ for 30min to replace the air in the flask. Under the N₂ protection, potassium hydroxide (0.3mol, 1eq) was added and the mixture was continuously stirred for 0.5h. Then cyclopentadiene monomers (0.8∼ 1.2eq) was added dropwise within 30min, and the mixture was continuously stirred for 1h under the N₂ protection. Then, the mixture was cooled to about -10°C; a mixed solution of n-hexane (150ml) and bromoethane (0.8∼1.2eq) was added dropwise into the three-necked flask for about 1 h, with the temperature controlled to be not higher than 20°C. And then the reaction continued for 1h or more in a self-heating process; then, diluted hydrochloric acid was added dropwise to adjust the pH to be acidic; the resulting mixture was stirred for 5-10min and then separated; the water layer was extracted with 150ml of n-hexane; the combined organic layers was washed by 250ml of water twice until the water phase becomes neutral, dried over 40g of anhydrous sodium sulfate and filtrated. Then the filtrate (about 400ml) was added into a 1,000ml three-necked flask and let standing for 7-20h at a temperature of 20±2°C to achieve isomerization wherein a GC was performed to determine the degree of isomerization, and ethyl cyclopentadiene was obtained.

### Step 2:

Under stirring, dichloroacetyl chloride (0.7∼1.5eq) was added into the n-hexane solution of the ethyl cyclopentadiene; after the mixture was cooled to about -15∼10°C, triethylamine (0.8∼1.5eq) was added dropwise for about 1 hr with the temperature controlled to be not greater than 15°C, and then the reaction proceeded for 1 h at a constant temperature; then, diluted hydrochloric acid was added dropwise to adjust the pH to be neutral or subacid; the resulting solution was stirred for 5-10min and separated, wherein concentration of the product in each of the resulting organic phase and the water phase was determined by TLC (with PE as a developing solvent). The organic phase was then subjected to rotary evaporation at a temperature of 35°C to remove solvent, and the residual liquid was dichlorobicycloketone.

### Step 3:

The dichlorobicycloketone prepared in the previous step, 125ml of tertiary butanol, 20ml of acetic acid and 25ml of water were added in sequence into a 1,000ml three-necked flask with a condenser pipe and heated to a temperature of 75∼80°C with stirring; 85ml (98%, 0.675mol) triethylamine was added dropwise (for about 1 h), and then the mixture was kept in a constant temperature overnight. The reaction was monitored by TLC with PE as a developing solvent. The resulting mixture was cooled to a temperature below 40°C. To the mixture were added 150ml of dichloromethane and diluted hydrochloric acid (10ml of concentrated hydrochloric acid +200ml of water) dropwise to adjust the pH to be acidic. After the pH adjustment, the solution was stirred for 5-10min and then separated, wherein TLC (developing solvent: EA:PA=1:5) result showed no presence of product in the resulting water layer. The organic phase was subjected to rotary evaporation to remove the solvent and then added into 200ml of petroleum ether. The mixture is stirred for half an hour and then let standing for separation into layers wherein the lower layer was black viscous liquid, and the upper layer was dark organic phase. The two layers were separated and the black viscous liquid was discarded. The organic phase (the upper layer) was added with 200ml of water and then followed by 150ml of aqueous solution of potassium hydroxide (3M) with stirring for extraction. The water layer was washed with petroleum ether. Then, the water layer was added with 200ml of petroleum ether, and diluted hydrochloric acid (3M) which was added into the mixture with stirring to adjust the pH to 1-2 for extraction. The organic layer was washed with water and subjected to rotary evaporation to remove the solvent and obtain a product, purity of which was 96% by HPLC. NMR result showed that the product was 4-ethyl-2-hydroxycyclohepta-2,4,6-trienone, a structural formula of which is: 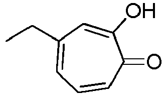 4-ethyl-2-hydroxycyclohepta-2,4,6-trienone: δH (DMSO, 500MHz) 7.271-7.315 (1 H,m), 7.144 (1H, s), 7.072-7.094(1H, d, J=11), 6.917-6.937(1 H,d,J=10), 2.564-2.609(2H,m), 1.146-1.176 (3H,m).

### Embodiment 6: Synthesis of IST_009_019

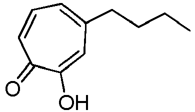

### Preparation of 4-butyl-2-hydroxycyclohepta-2,4,6-trienone

### Step 1:

At room temperature, to a 500ml three-necked flask was added 150ml of DMSO, stirred and purged with N₂ for 30min to replace the air in the flask. Under the N₂ protection, potassium hydroxide (0.3mol, 1eq) was added and the mixture was continuously stirred for 0.5h. Then cyclopentadiene monomers (0.8∼ 1.2eq) was added dropwise within 30min. The resulting mixture was continuously stirred for 1 h under the N₂ protection. Then, the mixture was cooled to about -10°C; a mixture of n-hexane (150ml) and n-butyl bromide (0.8∼1.2eq) was added dropwise into the three-necked flask for about 1 h, with the temperature controlled to be not higher than 20°C. And then the reaction continued for 1h or more in a self-heating process; then, diluted hydrochloric acid was added dropwise to adjust the pH to be acidic and the resulting mixture was fully stirred for 5-10min and then separated; the water layer was extracted with 150ml of n-hexane; the combined organic layers was washed with 250ml of water twice until the water phase became neutral, dried over 40g of anhydrous sodium sulfate and filtrated. Then the filtrate (about 400ml) was added into a 1,000ml three-necked flask and let standing for 7-20h at a temperature of 20±2°C to achieve isomerization wherein a GC was performed to determine the degree of isomerization, and n-butyl cyclopentadiene was obtained.

### Step 2:

Under stirring, dichloroacetyl chloride (0.7∼1.5eq) was added into the n-hexane solution of the n-butyl cyclopentadiene; after the mixture was cooled to about -15∼10°C, triethylamine (0.8∼1.5eq) was added dropwise within 1h with the temperature controlled to be not greater than 15°C, and then the reaction proceeded for 1h at a constant temperature; then, diluted hydrochloric acid was added dropwise to adjust the pH to be neutral or subacid; the solution was stirred for 5-10min and then separated, wherein concentration of the product in each of the resulting organic phase and the water phase was determined by TLC (with PE as a developing solvent). The organic phase was then subjected to rotary evaporation at a temperature of 35°C to remove solvent, and the residual liquid is dichlorobicycloketone.

### Step 3:

The dichlorobicycloketone prepared in the previous step, 125ml of tertiary butanol, 20ml of acetic acid and 25ml of water were added in sequence into a 1,000ml three-necked flask with a condenser pipe and heated to a temperature of 75∼80°C with stirring; 85ml (98%, 0.675mol) triethylamine was added dropwise within 1 hour, and then the mixture was stirred at a constant temperature overnight, and the reaction was monitored by TLC with PE as a developing solvent. At the completion of the reaction, the mixture was cooled to a temperature below 40°C. To the above mixture were added 200ml of ethyl acetate, 200ml of water and 10ml of concentrated hydrochloric acid for extraction. The organic layer was washed twice with 200ml of water and subjected to rotary evaporation to remove the solvent. The residue was distilled with a pressure of -0.1MPa and a external temperature of 150∼180°C. The fraction at 115-120°C were collected and 25g of product was obtained. The product is dissolved in the mixture of 150ml of n-hexane and 10ml of ethanol. The mixture was heated to 70 °C so that the product is completely dissolved. Then, the mixture was placed in a low-temperature tank cooled to a temperature of -5°C wherein a great amount of white crystals was observed and collected by filtration. The filter cake was washed with n-hexane to obtain a product. NMR result showed that the product was 4-butyl-2-hydroxycyclohepta-2,4,6-trienone, a structural formula of which is:

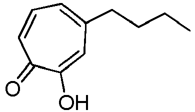

4-butyl-2-hydroxycyclohepta-2,4,6-trienone: δH (DMSO, 500 MHz) 7.267-7.309 (1 H, m), 7.142(1 H,s), 7.070-7.092 (1H, d, J=11), 6.910-6.931 (1H, d, J=10.5), 2.549-2.580(2H,m), 1.503-1.564(2H,m), 1.253-1.312(2H,m), 0.860-0.889(3H,m).

### Embodiment 7: Synthesis of IST_009_025

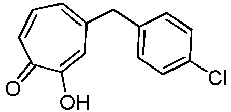

### Preparation of 4-(4-chlorobenzyl)-2-hydroxycyclohepta-2,4,6-trienone

### Step 1:

At room temperature, to a 500ml three-necked flask was added 150ml of DMSO, stirred and purged with N₂ for 30min to replace the air in the flask. Under the N₂ protection, potassium hydroxide (0.3mol, 1eq) was added and the mixture was continuously stirred for 0.5h. Then cyclopentadiene monomers (0.8∼ 1.2eq) were added dropwise within 30min, and the mixture was continuously stirred for 1 h under the N₂ protection. Then, the mixture was cooled to about -10°C; a mixed solution of n-hexane (150ml) and p-chlorobenzyl bromide (0.8∼1.2eq) was added dropwise into the three-necked flask for about 1 h, with the temperature controlled to be not higher than 20°C. And then the reaction continued for 1h or more in a self-heating process; then, diluted hydrochloric acid was added dropwise to adjust the pH to be acidic and the mixture was fully stirred for 5-10min and then separated; the water layer was extracted with 150ml of n-hexane; the combined organic layers are washed with 250ml of water twice until the water phase was neutral, dried over 40g of anhydrous sodium sulfate and filtrated. The filtrate (about 400ml) was added into a 1,000ml three-necked flask and let standing for 7-20h at a temperature of 20±2°C to achieve isomerization wherein a GC was performed to determine the degree of isomerization, and p-chlorobenzyl bromide cyclopentadiene was obtained.

### Step 2:

Under stirring, dichloroacetyl chloride (0.7∼1.5eq) was added into the n-hexane solution of the p-chlorobenzyl bromide cyclopentadiene; after the mixture was cooled to about -15∼10°C, triethylamine (0.8∼1.5eq) was added dropwise for about 1h with the temperature controlled to be not higher than 15°C, and then the reaction proceeded for 1h at a constant temperature; then, diluted hydrochloric acid was added dropwise to adjust the pH to be neutral or subacid; the solution was stirred for 5-10min and then separated, wherein concentration of the product in each of the resulting organic phase and the water phase was determined by TLC (with PE as a developing solvent). The organic phase was then subjected to rotary evaporation at a temperature of 35°C to remove solvent, and the residual liquid was dichlorobicycloketone.

### Step 3:

The dichlorobicycloketone prepared in the previous step, 125ml of tertiary butanol, 20ml of acetic acid and 25ml of water were added in sequence into a 1,000ml three-necked flask with a condenser pipe and heated to a temperature of 75∼80°C with stirring; 85ml (98%, 0.675mol) triethylamine was added dropwise within 1 hour, and then the resulting mixture was kept in a constant temperature overnight for reaction, and the reaction was monitored by TLC with PE as a developing solvent. At the completion of the reaction, the mixture was cooled to a temperature below 40°C. To the above reaction mixture were added with 100ml of dichloromethane, 200ml of water and 3ml of concentrated hydrochloric acid and separated into a water phase and an organic layer. The organic layer was washed twice with 100ml of water. The organic phase was added with 1 M aqueous solution of potassium hydroxide for extraction. The water phase was washed twice with 100ml of dichloromethane. Then the water phase was added with 100ml of dichloromethane, and 2M diluted hydrochloric acid until it was acidic. The mixture was subject to extraction and separated. The organic phase was washed twice with distilled water and subjected to rotary evaporation to obtain a product, purity of which was 99% by HPLC. NMR result showed that the 4-(4-chlorobenzyl)-2-hydroxycyclohepta-2,4,6-trienone, a structural formula of which is:

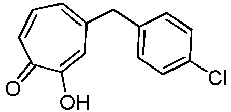

4-(4-chlorobenzyl)-2-hydroxycyclohepta-2,4,6-trienone: δH (DMSO, 500 MHz) 7.304-7.323 (2H,d,J=9.5), 7.213-7.284(1 H,m), 7.183(1 H,s), 7.157-7.139(2H,d,J=9.5), 7.071-7.049(1H, d, J=11), 6.986-7.006(1 H,d,J=10), 3.953(2H, s).

### Embodiment 8: Synthesis of IST_009_028

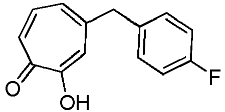

### Preparation of 4-(4-fluorobenzyl)-2-hydroxycyclohepta-2,4,6-trienone

### Step 1:

At room temperature, to a 500ml three-necked flask was added 150ml DMSO is added, stirred and purged with N₂ for 30min to replace the air in the flask. Under the N₂ protection, potassium hydroxide (0.3mol, 1eq) was added and the mixture was continuously stirred for 0.5h. Then cyclopentadiene monomers (0.8∼1.2eq) was added dropwise within 30min, and the mixture was continuously stirred for 1 h under the N₂ protection. Then, the mixture was cooled to about -10°C. To the above mixture was added a mixed solution of n-hexane (150ml) and p-fluorobenzyl bromide (0.8∼1.2eq)within 1h at the temperature controlled to be not higher than 20°C. And then the reaction continued for 1h or more in a self-heating process; then, diluted hydrochloric acid was added dropwise to adjust the pH to be acidic and the mixture was fully stirred for 5-10min and then separated; the water layer was extracted one more time with 150ml of n-hexane; the combined organic layers were washed with 250ml of water twice until the water phase was neutral, dried over 40g of anhydrous sodium sulfate and filtrated. And then the filtrate (about 400ml) is added into a 1,000ml three-necked flask and let standing for 7-20h at a temperature of 20±2°C to achieve isomerization wherein a GC was performed to determine the degree of isomerization, and p-fluorobenzyl bromide cyclopentadiene was obtained.

### Step 2:

Under stirring, dichloroacetyl chloride (0.7∼1.5eq) was added into the n-hexane solution of the p-fluorobenzyl bromide cyclopentadiene; after the mixture was cooled to about -15∼10°C, triethylamine (0.8∼1.5eq) was added dropwise within 1h with the temperature controlled to be not higher than 15°C, and then the reaction proceeded for 1h at a constant temperature; then, diluted hydrochloric acid was added dropwise to adjust the pH to be neutral or subacid; the resulting solution was stirred for 5-10min and then separated, wherein concentration of the product in each of the resulting organic phase and the water phase was determined by TLC (with PE as a developing solvent). The organic phase was subjected to rotary evaporation at a temperature of 35°C to remove solvent, and the residual liquid is dichlorobicycloketone.

### Step 3:

The dichlorobicycloketone prepared in the previous step, 125ml of tertiary butanol, 20ml of acetic acid and 25ml of water were added in sequence into a 1,000ml three-necked flask with a condenser pipe and heated to a temperature of 75∼80°C with stirring; 85ml (98%, 0.675mol) triethylamine was added dropwise within 1 hour, and then the mixture was kept in a constant temperature overnight, and the reaction was monitored by TLC with PE as a developing solvent. At the completion of the reaction, the resulting mixture was cooled to a temperature below 40°C and added with 100ml of dichloromethane, 200ml of water and 3ml of concentrated hydrochloric acid for extraction and thereafter separate into a water phase and an organic layer. The organic layer was washed twice with 100ml of water. The organic phase was added with 1 M aqueous solution of potassium hydroxide for extraction and separated. The water phase was washed twice with 100ml of dichloromethane. Then the water phase was added with 100ml of dichloromethane, and 2M diluted hydrochloric acid until it was acidic. The mixture was separated, and the organic phase was washed twice with distilled water and subjected to rotary evaporation to obtain a crude product,

The crude product was dissolved in about 50ml of ethanol. The mixture was placed in a refrigerator overnight wherein solids was observed and collected by filtration. The filter cake was washed with about 5-10ml of ethanol. The filtrate was subjected to rotary evaporation to obtain a product, the purity of which was 98% by HPLC. NMR result shows that the product was 4-(4-fluorobenzyl)-2-hydroxycyclohepta-2,4,6-trienone, a structural formula of which is:

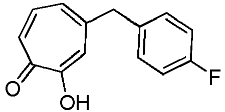

4-(4-fluorobenzyl)-2-hydroxycyclohepta-2,4,6-trienone: δH (DMSO, 500MHz) 7.306-7.325(2H,d,J=9.5), 7.183-7.285 (1H, m), 7.157 (1H, s), 7.120-7.139(2H,d,J=9.5), 7.067-7.089 (1H, d, J=11), 6.973-6.993 (1H,d,J=10), 3.946(2H, s).

### Embodiment 9: Synthesis of IST_009_030 (2-hydroxy-4-n-octyl-2,4,6-cycloheptatriene-1-ketone)

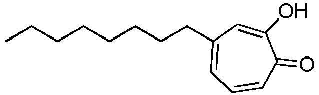

### Preparation of 2-hydroxy-4-octylcyclohepta-2,4,6-trienone

### Step 1:

At room temperature, to a 500ml three-necked flask was added 150ml DMSO is added, stirred and purged with N₂ for 30min to replace the air in the flask. Under the N₂ protection, potassium hydroxide (0.3mol, 1eq) was added and the mixture was continuously stirred for 0.5h. Then cyclopentadiene monomers (0.8∼1.2eq) was added dropwise within 30min, and the resulting mixture was continuously stirred for 1 h under the N₂ protection. Then, the mixture was cooled to about -10°C. To the above reaction mixture was added a mixed solution of n-hexane (150ml) and 1-bromooctane (0.8∼1.2eq) within 1 h, with the temperature controlled to be not higher than 20°C. And then the reaction continued for 1h or more in a self-heating process; then, diluted hydrochloric acid was added dropwise to adjust the pH to be acidic; after the adjustment was completed, the mixture was fully stirred for 5-10min and then separated; the water layer was extracted one more time with 150ml of n-hexane; the combined organic layers were washed with 250ml of water twice until the water phase was neutral, dried over 40g of anhydrous sodium sulfate and filtrated, and then the filtrate (about 400ml) is added into a 1,000ml three-necked flask and let standing for 7-20h at a temperature of 20±2°C to achieve isomerization wherein a GC was performed to determine the degree of isomerization, and n-octyl cyclopentadiene was obtained.

### Step 2:

Under stirring, dichloroacetyl chloride (0.7∼1.5eq) was added into the n-hexane solution of the n-octyl cyclopentadiene; after the mixture was cooled to about -15∼10°C, triethylamine (0.8∼1.5eq) was added dropwise for about 1hr with the temperature controlled to be not higher than 15°C, and then the reaction proceeded for 1h at a constant temperature; then, diluted hydrochloric acid was added dropwise to adjust the pH to be neutral or subacid; the solution was stirred for 5-10min and then separated, wherein concentration of the product in each of the resulting organic phase and the water phase was determined by TLC (with PE as a developing solvent). The organic phase is then subjected to rotary evaporation at a temperature of 35°C to remove solvent, and the residual green liquid is dichlorobicycloketone.

### Step 3:

The dichlorobicycloketone prepared in the previous step, 125ml of tertiary butanol, 20ml of acetic acid and 25ml of water were added in sequence into a 1,000ml three-necked flask with a condenser pipe and heated to a temperature of 75∼80°C with stirring; 85ml (98%, 0.675mol) triethylamine was added dropwise within 1 hour, and then the mixture was kept in a constant temperature overnight for reaction, and the reaction was monitored by TLC with PE as a developing solvent. At the completion of the reaction, the mixture was cooled to a temperature below 40°C. To the above reaction mixture was added 300ml of dichloromethane resulting in precipitation of a great amount of solids, The solids were separated from the liquid, washed with petroleum ether, and then filtrated; the combined organic phases was added with about 200ml of distilled water for extraction and thereafter separated. To the organic phase (the upper layer) was added 200ml of water, and 150ml of 3M aqueous solution of potassium hydroxide with stirring for extraction. The water layer was washed with petroleum ether. Then, into the water layer was added another 200ml of petroleum ether and 3M diluted hydrochloric acid to adjust the pH value to 1-2 for extraction and thereafter separated. The organic layer was washed with water, and then separated with silica gel chromatography to obtain a product, the purity of which was 99% by HPLC. NMR result showed that the product was 2-hydroxy-4-octylcyclohepta-2,4,6-trienone, a structural formula of which is:

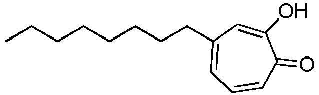

2-hydroxy-4-octylcyclohepta-2,4,6-trienone: δH (DMSO, 500 MHz) 7.250-7.273 (1 H,m), 7.133(1 H,s), 7.062-7.083 (1H, d, J=10.5), 6.097-6.927 (1H, d, J=10), 2.556-2.571 (2H,m), 1.530-1.544(2H,m), 1.214-1.247(10H,m), 0.814-0.841(3H,m).

### Embodiment 10: Synthesis of IST_009_031

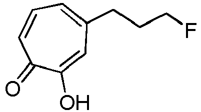

### Preparation of 4-(3-fluoropropyl)-2-hydroxycyclohepta-2,4,6-trienone

### Step 1:

At room temperature, to a 500ml three-necked flask was added 150ml DMSO is added, stirred and purged with N₂ for 30min to replace the air in the flask. Under the N₂ protection, potassium hydroxide (0.3mol, 1eq) was added and the mixture was continuously stirred for 0.5h. Then cyclopentadiene monomers (0.8∼1.2eq) was added dropwise within 30min, and the resulting mixture was continuously stirred for 1 h under the N₂ protection. Then, the mixture was cooled to about -10°C and a mixed solution of n-hexane (150ml) and 1-fluoro-3-bromopropane (0.8∼1.2eq) was added dropwise into the three-necked flask for about 1h, with the temperature controlled to be not higher than 20°C. And then the reaction continued for 1h or more in a self-heating process; then, diluted hydrochloric acid was added dropwise to adjust the pH to be acidic and the mixture was fully stirred for 5-10min and then separated; the water layer was extracted one more time with 150ml of n-hexane; the combined organic layers were washed with 250ml of water twice until the water phase was neutral, dried over 40g of anhydrous sodium sulfate and filtrated. And then the filtrate (about 400ml) was added into a 1,000ml three-necked flask and let standing for 7-20h at a temperature of 20±2°C to achieve isomerization wherein a GC was performed to determine the degree of isomerization, and 3-fluoropropyl cyclopentadiene was obtained.

### Step 2:

Under stirring, dichloroacetyl chloride (0.7∼1.5eq) was added into the n-hexane solution of the 3-fluoropropyl cyclopentadiene; after the mixture was cooled to about -15∼10°C, triethylamine (0.8∼1.5eq) was added dropwise for about 1 hr at the temperature controlled to be not higher than 15°C, and then the reaction proceeded for 1h at a constant temperature; then, diluted hydrochloric acid was added dropwise to adjust the pH to be neutral or subacid; the solution was stirred for 5-10min and then separated, wherein concentration of the product in each of the resulting organic phase and the water phase was determined by TLC (with PE as a developing solvent). The organic phase was then subjected to rotary evaporation at a temperature of 35°C to remove solvent, and the residual liquid is dichlorobicycloketone.

### Step 3:

The dichlorobicycloketone prepared in the previous step, 125ml of tertiary butanol, 20ml of acetic acid and 25ml of water were added in sequence into a 1,000ml three-necked flask with a condenser pipe and heated to a temperature of 75∼80°C with stirring; 85ml (98%, 0.675mol) triethylamine was added dropwise (for about 1 h), and then the mixture was kept at a constant temperature overnight for reaction, and the reaction was monitored by TLC with PE as a developing solvent. At the completion of the reaction, the mixture was cooled to a temperature below 40°C. To the above reaction mixture were added 200ml of ethyl acetate, 200ml of water and 10ml of concentrated hydrochloric acid for extraction and thereafter separated. The organic layer was washed twice with 200ml of water and subjected to rotary evaporation to remove the solvent. The residue was subject to decompression distillation with a pressure of -0.1MPa and a external temperature of 140∼150°C. The fraction at 130-135°C was collected to give product as yellow liquid. NMR result showed that the product is 4-(3-fluoropropyl)-2-hydroxycyclohepta-2,4,6-trienone.

4-(3-fluoropropyl)-2-hydroxycyclohepta-2,4,6-trienone: δH (DMSO, 500 MHz) 7.277-7.319(1 H, m), 7.170(1 H, s), 7.083-7.105(1H, d, J=11), 6.928-6.948(1H, d, J=10), 4.484-4.508 (1H,m), 4.390-40401 (1 H, m), 2.650-2.681 (2H,m), 1.892-1.997(2H,m).

### Example 11: Synthesis of IST_009_033

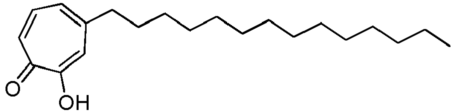

### Preparation of 2-hydroxy-4-tetradecylcyclohepta-2,4,6-trienone

### Step 1:

At room temperature, to a 500ml three-necked flask was added 150ml DMSO is added , stirred and purged with N₂ for 30min to replace the air in the flask. Under the N₂ protection, potassium hydroxide (0.3mol, 1eq) was added and the mixture was continuously stirred for 0.5h. Then cyclopentadiene monomers (0.8∼1.2eq) was added dropwise within 30min, and the resulting mixture was continuously stirred for 1 h under the N₂ protection. Then, the mixture was cooled to about -10°C. To the above mixture was added a mixed solution of n-hexane (150ml) and bromotetradecane (0.8∼1.2eq) dropwise within 1h at the temperature controlled to be not higher than 20°C. And then the reaction continued for 1h or more in a self-heating process; then, diluted hydrochloric acid was added dropwise to adjust the pH to be acidic and the mixture was fully stirred for 5-10min and then separated; the water layer was extracted one more time with 150ml of n-hexane; the combined organic layers were washed with 250ml of water twice until the water phase was neutral, dried over 40g of anhydrous sodium sulfate and filtrated. And then the filtrate (about 400ml) was added into a 1,000ml three-necked flask and let standing for 7-20h at a temperature of 20±2°C to achieve isomerization wherein a GC was performed to determine the degree of isomerization, and n-tetradecyl cyclopentadiene was obtained.

### Step 2:

Under stirring, dichloroacetyl chloride (0.7∼1.5eq) was added into the n-hexane solution of the n-tetradecyl cyclopentadiene; after the mixture was cooled to about -15∼10°C, triethylamine (0.8∼1.5eq) was added dropwise for about 1 hr at the temperature controlled to be not higher than 15°C, and then the reaction proceeded for 1h at a constant temperature; then, diluted hydrochloric acid was added dropwise to adjust the pH to be neutral or subacid; the solution was stirred for 5-10min and then separated, wherein concentration of the product in each of the resulting organic phase and the water phase was determined by TLC (with PE as a developing solvent). The organic phase was then subjected to rotary evaporation at a temperature of 35°C to remove solvent, and the residual liquid was dichlorobicycloketone.

### Step 3:

The dichlorobicycloketone prepared in the previous step, 125ml of tertiary butanol, 20ml of acetic acid and 25ml of water were added in sequence into a 1,000ml three-necked flask with a condenser pipe and heated to a temperature of 75∼80°C with stirring; 85ml (98%, 0.675mol) triethylamine was added dropwise (for about 1 h), and then the mixture was kept in a constant temperature overnight for reaction, and the reaction was monitored by TLC with PE as a developing solvent. At the completion of the reaction, the mixture was cooled to a temperature below 40°C. To the resulting mixture were added 100ml of petroleum ether and 100ml of water for extraction and thereafter separated. The organic phase was mixed with silica gel and then subjected to column chromatography(PE/EA=10:1) to give the product. NMR result showed that the product was 2-hydroxy-4-tetradecylcyclohepta-2,4,6-trienone, a structural formula of which is: 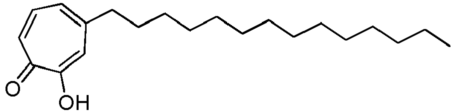 2-hydroxy-4-tetradecylcyclohepta-2,4,6-trienone: δH (DMSO, 500 MHz) 7.273-7.340(1 H, m), 7.145(1 H,s), 7.073-7.095(1H, d, J=11), 6.920-6.940(1H, d, J=10), 2.506-2.587(2H,m), 1.561 (2H, m), 1.227-1.275(22H,m), 0.835-0.861 (3H,m).

### Example 12: In vitro antibacterial activity (MICs) of β-hinokitiol derivatives

Doubling dilutions were performed to determine the minimum inhibitory concentration of β-hinokitiol derivatives against different bacteria in vitro. Tables 1 to 4 were results of the test. Tables 1 to 4 showed that the β-hinokitiol derivatives had broad-spectrum antibacterial activities and showed high inhibitory activities of on major animal pathogenic bacteria (for example, clostridium perfringens, staphylococcus aureus, escherichia coli, salmonella, riemerella anatipestifer, haemophilus parasuis, vibrio and streptococcus), and in particular that, the antibacterial activities of additives such as IST_009_001 and IST_009_003 were better than those of the lead compound IST_001_002 (β-hinokitiol).

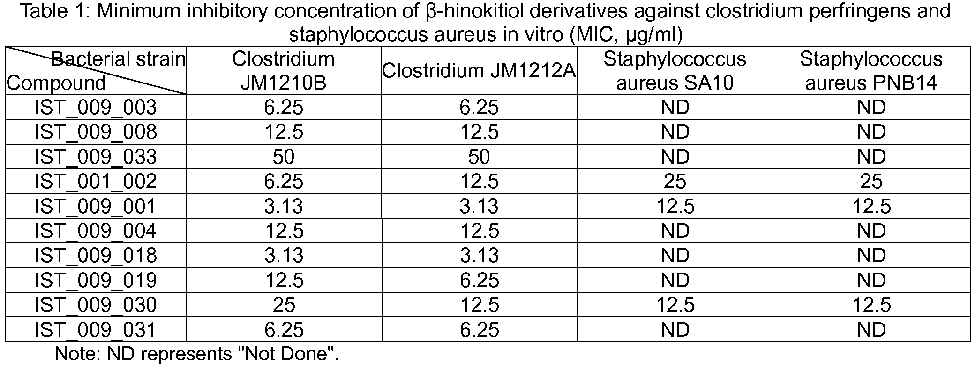

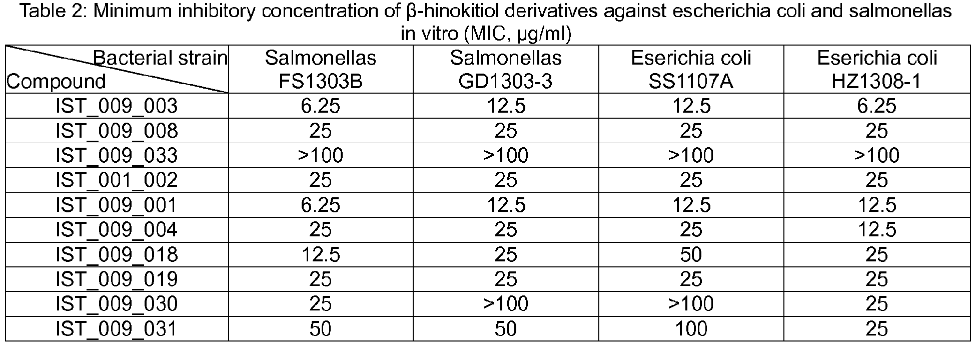

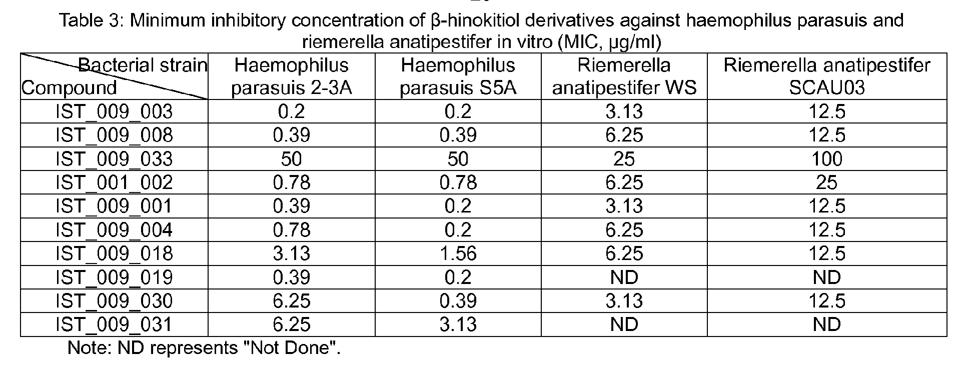

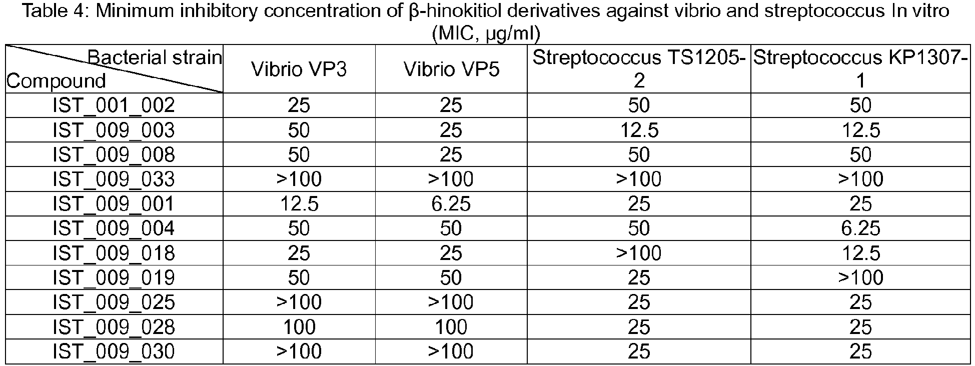

### Example 13: Application effects of β-hinokitiol derivatives in broiler feeds

598 fast-grown type yellow feather broilers (female) aged 30-days were divided into five groups according to table 5. Each group was respectively added with β-hinokitiol derivatives or/and colistin sulfate. During the test, the broilers ate food and drank water freely. The test lasted for 14 days. Results showed that β-hinokitiol derivatives such as IST_009_001 and IST_009_003 could enhance the weight gain and the feed conversion efficiency of the experimental broilers (table 6).

**Table 5: Grouping of application effect test of β-hinokitiol derivatives in broiler feeds**

| Group | Quantity of the broilers | Additives | Dosage (ppm) | Administration |
|---|---|---|---|---|
| 1 | 39 | - | - | Mixed with feed |
| 2 | 40 | IST_009_001 | 5 | Mixed with feed |
| 3 | 40 | IST_009_001 | 15 | Mixed with feed |
| 4 | 40 | IST_009_003 | 40 | Mixed with feed |
| 5 | 40 | IST_009_001+colistin sulfate | 10+20 | Mixed with feed |
| 6 | 40 | - | - | Mixed with feed |
| 7 | 40 | IST_009_001 | 5 | Mixed with feed |
| 8 | 40 | IST_009_001 | 15 | Mixed with feed |
| 9 | 40 | IST_009_003 | 40 | Mixed with feed |
| 10 | 40 | IST_009_001+colistin sulfate | 10+20 | Mixed with feed |
| 11 | 40 | - | - | Mixed with feed |
| 12 | 40 | IST_009_001 | 5 | Mixed with feed |
| 13 | 40 | IST_009_001 | 15 | Mixed with feed |
| 14 | 39 | IST_009_003 | 40 | Mixed with feed |
| 15 | 40 | IST_009_001+colistin sulfate | 10+20 | Mixed with feed |

**Table 6: Results of application effect test of β-hinokitiol derivatives in broiler feeds**

| Group | Additives | Dosage (ppm) | Average weight gain (g) | Total consumption (kg) | Average daily weight gain (g/d*bird) | feed conversion efficiency |
|---|---|---|---|---|---|---|
| 1 | - | - | 475.16 | 54.67 | 32.01 | 3.049 |
| 2 | IST_009_001 | 5 | 547.21 | 55.12 | 33.52 | 2.808 |
| 3 | IST_009_001 | 15 | 523.38 | 57.84 | 34.53 | 2.889 |
| 4 | IST_009_003 | 40 | 502.05 | 58.05 | 36.21 | 2.862 |
| 5 | 1ST 009 001+colistin sulfate | 10+20 | 528.50 | 57.63 | 36.82 | 2.795 |
| 6 | - | - | 517.00 | 60.27 | 36.67 | 2.812 |
| 7 | IST_009_001 | 5 | 516.79 | 61 | 37.75 | 2.886 |
| 8 | IST_009_001 | 15 | 517.17 | 58.03 | 36.94 | 2.807 |
| 9 | IST_009_003 | 40 | 449.90 | 57.71 | 34.19 | 2.952 |
| 10 | 1ST 009 001+colistin sulfate | 10+20 | 507.00 | 60.39 | 38.18 | 2.825 |
| 11 | - | - | 507.24 | 60.55 | 35.13 | 2.980 |
| 12 | IST_009_001 | 5 | 520.00 | 58.85 | 36.93 | 2.846 |
| 13 | IST_009_001 | 15 | 525.50 | 57.61 | 37.5 | 2.741 |
| 14 | IST_009_003 | 40 | 534.50 | 58.17 | 37.14 | 2.868 |
| 15 | IST_009_001+colistin sulfate | 10+20 | 527.00 | 56.61 | 37.64 | 2.685 |

### Example 14: Effect of β-hinokitiol derivatives on diarrhea of weaned pigs

80 25-day-old weaned pigs were randomly divided into eight groups according to table 7, 10 pigs in each group. The basic diets without any antibiotics were continuously added with IST_009_001, colistin sulfate or IST_001_002 according to the dosage in the table. During the testing, the pigs ate food and drank water freely with temperature preservation. The test lasted for 10 days. Results (table 7) showed that, adding IST_009_001 in the feeds could obviously reduce the diarrhea rate of the tested pigs, and the effect of the IST_009_001 30ppm test group was obviously better than that of colistin sulfate or IST_001_002 groups.

**Table 7: Effect of β-hinokitiol derivatives on diarrhea of weaned pigs**

| Group | Additives | Dosage (ppm) | Average daily diarrhea rate (%) |
|---|---|---|---|
| 1 | Blank group | 0 | 52 |
| 2 | Blank group | 0 | 57 |
| 3 | Colistin sulfate | 60 | 30 |
| 4 | Colistin sulfate | 60 | 33 |
| 5 | IST_009_001 | 30 | 16 |
| 6 | IST_009_001 | 30 | 17 |
| 7 | IST_001_002 | 30 | 35 |
| 8 | IST_001_002 | 30 | 32 |

### Embodiment 15: Protection effect of β-hinokitiol derivatives against attacking and infection of riemerella anatipestifer

100 16-day-old cherry valley ducks were randomly divided into 10 test groups according to table 8. Each duck was injected with riemerella anatipestifer of 5×10⁷CFU via intramuscular injection for the purpose of attacking and infection, and injected with the medicines via intramuscular injection simultaneously. Morbidity and death of the tested ducks were observed, and dead ducks were subject to autopsy to verify whether the death was caused by riemerella anatipestifer infection. The observation lasted for 7 days. All tested ducks were executed at the end of the test. Autopsy were performed to confirm the morbidity. The statistic results can be seen in table 8. Table 8 showed that the β-hinokitiol derivative IST_009_001 could protect ducks against attacking and infection of riemerella anatipestifer.

**Table 8: Protection effect of β-hinokitiol derivatives against attacking and infection of riemerella anatipestifer**

| Group | Quantity of the ducks | Dosage of riemerella anatipestifer (CFU/bird) | Medicine | Dosage (mg/kg weight) | Morbidity (%) | Death rate (%) |
|---|---|---|---|---|---|---|
| 1 | 20 | - | - | - | 0 | 0 |
| 2 | 20 | 5x10⁷ | - | - | 100 | 65 |
| 3 | 20 | 5x10⁷ | Cefotaxime | 10 | 25 | 0 |
| 4 | 20 | 5x10⁷ | IST_009_001 | 10 | 40 | 0 |
| 5 | 20 | 5x10⁷ | IST_009_001 | 20 | 25 | 0 |

## Claims

1. β-hinokitiol derivatives, having a structural formula as shown in formula (I): 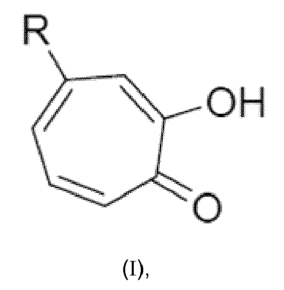 wherein, R is n-propyl, n-pentyl, benzyl, sec-butyl, ethyl, n-butyl, p-chlorobenzyl, p-fluorobenzyl, n-octyl, 1-fluoropropyl or n-tetradecyl.

2. Application of any one of the β-hinokitiol derivatives according to claim 1 in preparation of animal antibacterial agents and antibacterial growth promoters used in feed.

3. The application according to claim 2, wherein said animals include pigs, chickens, ducks, geese, beef cattle, dairy cattle, sheep, fish, shrimp, foxes, martens or raccoon dogs in all growth stages.

4. The application according to claim 2, wherein an additive dosage of said β-hinokitiol derivative in animal feeds is 0.1 1∼200ppm.

5. The application according to claim 2, wherein, when the animal antibacterial agents are used to treat diseases caused by infection with various animals sensitive bacteria, the dosage is 1-30mg/kg body weight.
